# EUROPEAN PATENT APPLICATION

(11) **EP 4 678 728 A1**
(43) Date of publication of application: **14.01.2026**
(21) Application number: 24770797.9
(22) Date of filing: 08.03.2024
(51) Int. Cl.: C12M 3/00

(54) **CULTURE VESSEL**

(30) Priority: 10.03.2023 JP 2023037896
(71) Applicant: NOK Corporation, Minato-ku Tokyo 105-8585 (JP); NATIONAL UNIVERSITY CORPORATION YOKOHAMA NATIONAL UNIVERSITY, Yokohama-shi Kanagawa 240-8501 (JP)
(72) Inventor: FUTASHIMA, Ryo, Fujisawa-shi, Kanagawa 251-0042 (JP); FUNAHASHI, Akira, Fujisawa-shi, Kanagawa 251-0042 (JP); YAMAMOTO, Hiroki, Fujisawa-shi, Kanagawa 251-0042 (JP); UDA, Toru, Fujisawa-shi, Kanagawa 251-0042 (JP); FUKUDA, Junji, Yokohama-shi, Kanagawa 240-8501 (JP)
(74) Representative: Global IP Europe Patentanwaltskanzlei
(86) International application number: PCT/JP2024/009155
(87) International publication number: WO 2024/190691

(57) **Abstract**

A culture vessel (1) includes: a rigid member (2) that is a member that does not deform when grasped for handling; and a bottom member (3) that is a member having oxygen supply performance for culturing an object to be cultured.

## Description

### Technical Field

The present invention relates to a culture vessel.

### Background Art

Cultured cells are widely used in fields such as medicine and pharmaceuticals. Spheroids that are clusters of aggregated cells are expected to be used in drug screening, regenerative medicine, and the like because spheroids exhibit functions similar to those of cells, tissues, organs, and the like in a living body. For example, a culture vessel on which a large number of recesses (dimples) are formed is used to culture spheroids. Cells are seeded into each recess, cultured, and aggregated within each depression to form spheroids (for example, refer to Patent Literature 1).

### Citation List

### Patent Literature

Patent Literature 1: International Publication No. WO 2017/073625

### Summary of Invention

### Technical Problem

In aerobic cells, spheroid culture requires more oxygen than individual cell culture, and if sufficient oxygen is not supplied, the cells may become necrotic. Therefore, culture vessels used for spheroid culture require high oxygen supply performance, and materials with high oxygen permeability are used as materials of spheroid culture vessels. On the other hand, culture vessels must be easy to handle. As described above, there is a need for a configuration that enables oxygen supply performance to be increased and handling to be made easier with respect to conventional spheroid culture vessels.

The present invention has been made in consideration of the problem described above and an object thereof is to provide a culture vessel that enables oxygen supply performance to be increased and handling to be made easier.

### Solution to Problem

In order to achieve the object described above, a culture vessel according to the present invention includes: a member that does not deform when grasped for handling; and a bottom member that is a member having oxygen supply performance for culturing an object to be cultured.

In a culture vessel according to an aspect of the present invention, an oxygen permeation coefficient of the bottom member is 20 × 10⁻¹³ cm³ (STP) × cm/(cm² × s × Pa) or more.

In the culture vessel according to an aspect of the present invention, the bottom member is formed from a silicone resin.

In the culture vessel according to an aspect of the present invention, a flexural modulus of the member that does not deform is 500 MPa or more.

In the culture vessel according to an aspect of the present invention, the member that does not deform is formed from a hard resin.

In the culture vessel according to an aspect of the present invention, the bottom member is attached to the member that does not deform.

The culture vessel according to an aspect of the present invention includes a plurality of tubular portions being portions that have a tubular shape and a support portion being a portion that supports the plurality of tubular portions, wherein the member that does not deform includes at least the support portion among the plurality of tubular portions and the support portion, and the bottom member closes one opening of each of the plurality of tubular portions.

In the culture vessel according to an aspect of the present invention, a bottom surface portion being a portion of the bottom member that closes the one opening of each of the plurality of tubular portions has a smooth surface that closes the opening.

In the culture vessel according to an aspect of the present invention, a plurality of recessed portions are formed on the bottom surface portion being a portion of the bottom member that closes the one opening of each of the plurality of tubular portions.

In the culture vessel according to an aspect of the present invention, an opening of each recessed portion reduces in diameter toward a bottom of the recessed portion.

In the culture vessel according to an aspect of the present invention, a cross section of the opening taken along a surface along a recess direction of the recessed portion draws an arc.

In the culture vessel according to an aspect of the present invention, a bottom of the recessed portion forms a surface along a spherical surface.

In the culture vessel according to an aspect of the present invention, in the bottom surface portion, the opening of one of the recessed portions contacts the opening of another recessed portion in proximity.

In the culture vessel according to an aspect of the present invention, a space between the opening and the bottom of the recessed portion is a tubular surface.

In the culture vessel according to an aspect of the present invention, a depth of the recessed portion is 0.5 mm or more and 2.0 mm or less.

In the culture vessel according to an aspect of the present invention, the member that does not deform includes the plurality of tubular portions and the support portion.

In the culture vessel according to an aspect of the present invention, the silicone resin is polydimethylsiloxane.

The culture vessel according to an aspect of the present invention is used for culturing spheroids and the object to be cultured is cells.

### Advantageous Effect of Invention

The culture vessel according to the present invention enables oxygen supply performance to be increased, handling to be made easier, and fine structures to be provided.

### Brief Description of Drawings

[Figure 1] Figure 1 is a perspective view of a culture vessel according to an embodiment of the present invention.
[Figure 2] Figure 2 is another perspective view of the culture vessel according to the embodiment of the present invention.
[Figure 3] Figure 3 is a plan view of the culture vessel illustrated in Figure 1.
[Figure 4] Figure 4 is a sectional view of the culture vessel taken along a plane along a line A-A in Figure 3.
[Figure 5] Figure 5 is a sectional view illustrating, enlarged, one tubular portion and a vicinity thereof illustrated in Figure 4.
[Figure 6] Figure 6 is a sectional perspective view illustrating, enlarged, a vicinity of a bottom surface portion of a bottom member.
[Figure 7] Figure 7 is a sectional view illustrating a cross section of the bottom surface portion of the bottom member taken along a plane including an axial line of a tubular portion.
[Figure 8] Figure 8 is a sectional view illustrating, enlarged, a recessed portion illustrated in Figure 7.
[Figure 9] Figure 9 is a sectional view illustrating, enlarged, one tubular portion and a vicinity thereof illustrated in Figure 4 and is a sectional view illustrating a modification of the bottom surface portion of the bottom member.

### Description of Embodiment

Hereinafter, an embodiment of the present invention will be described with reference to the drawings.

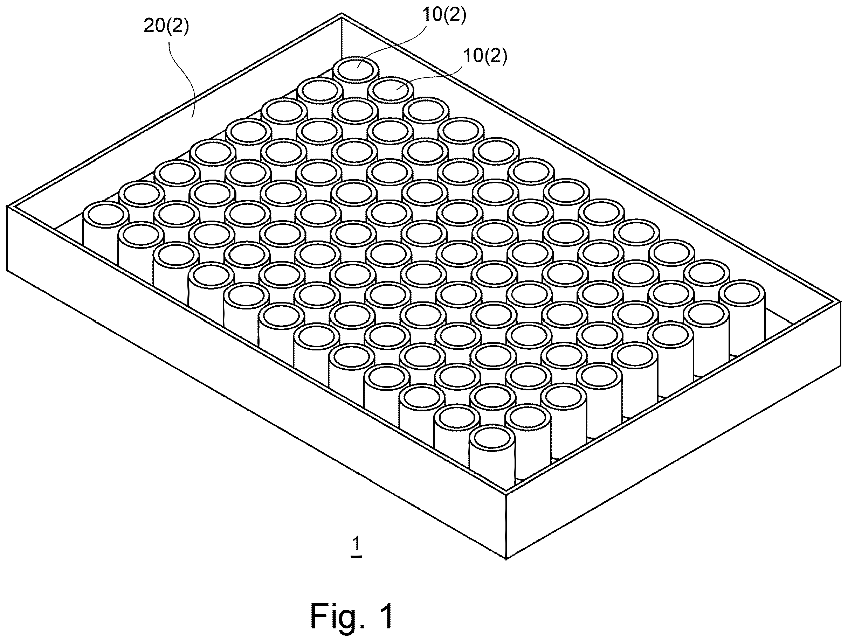

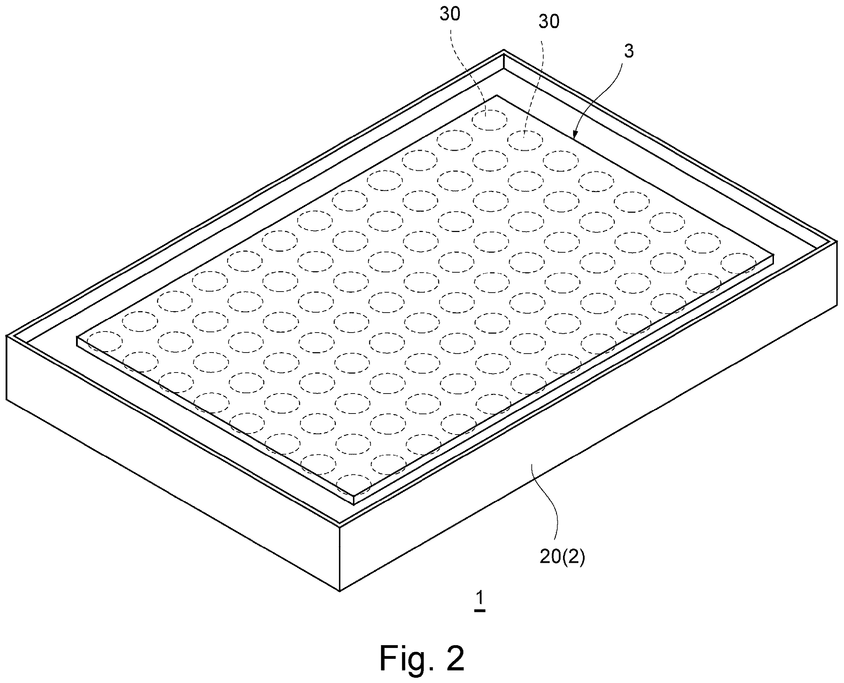

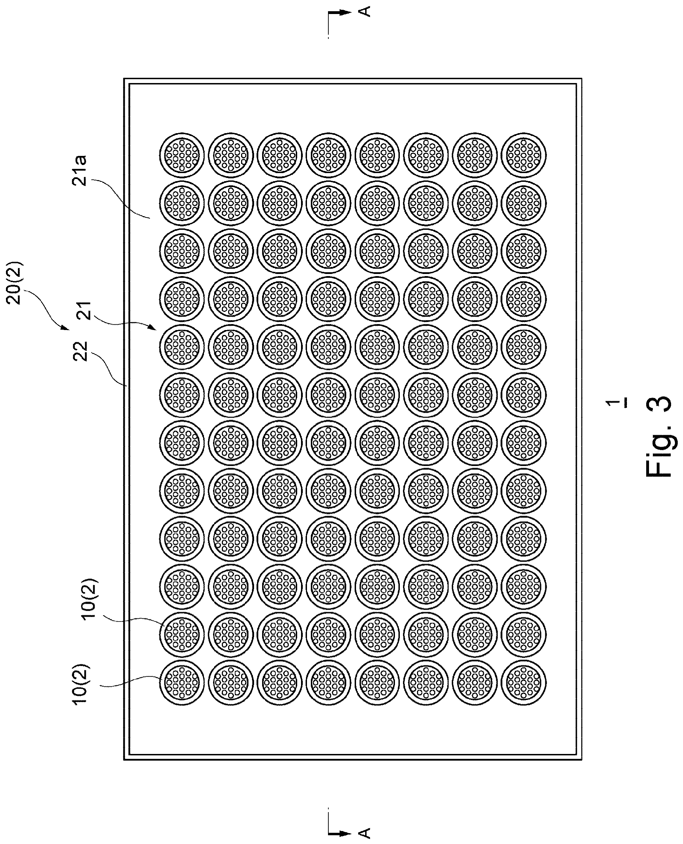

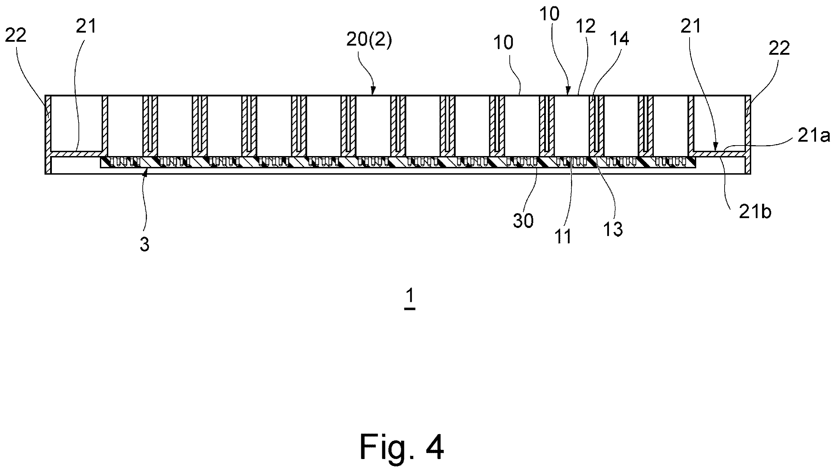

Figure 1 is a perspective view of a culture vessel 1 according to an embodiment of the present invention, Figure 2 is another perspective view of the culture vessel 1, Figure 3 is a plan view of the culture vessel 1, and Figure 4 is a sectional view taken along a plane along a line A-A in Figure 3 of the culture vessel 1. The culture vessel 1 is used to culture an object to be cultured. The object to be cultured is, for example, cells. Hereinafter, the culture vessel 1 is to be used to culture spheroids. Note that applications of the culture vessel 1 are not limited to culturing spheroids and the culture vessel 1 is also used to culture other objects to be cultured.

As illustrated in Figures 1 to 4, the culture vessel 1 includes: a rigid member 2 being a member that does not deform when grasped for handling; and a bottom member 3 being a member that has oxygen supply performance for culturing an object to be cultured. Hereinafter, a configuration of the culture vessel 1 will be specifically described. Note that in the drawings, reference signs are not assigned to all of the plurality of members and reference signs may be omitted.

The rigid member 2 is formed from, for example, a hard resin. The bottom member 3 is attached to the rigid member 2. As illustrated in Figures 1 to 4, the culture vessel 1 includes a plurality of tubular portions 10 being portions that have a tubular shape and a support portion 20 being a portion that supports the plurality of tubular portions 10. The rigid member 2 includes at least the support portion 20 among the plurality of tubular portions 10 and the support portion 20. In the culture vessel 1 according to the present embodiment, the rigid member 2 includes the plurality of tubular portions 10 and the support portion 20. The rigid member 2, for example, is a well plate.

As illustrated in Figures 3 and 4, for example, the support portion 20 has a supporting plate portion 21 and a side wall portion 22. The supporting plate portion 21 is a portion that spreads in a plate shape and has an upper surface 21a and a lower surface 21b that constitute a pair of surfaces. In addition, the supporting plate portion 21 supports each of the plurality of tubular portions 10. As illustrated in Figures 3 and 4, the supporting plate portion 21 spreads between adjacent tubular portions 10 and also spreads between the tubular portions 10 and the side wall portion 22. Furthermore, the tubular portions 10 penetrate the supporting plate portion 21. The side wall portion 22 is an annular portion with a plate shape that encloses a periphery of the supporting plate portion 21. The side wall portion 22 encloses the plurality of tubular portions 10. The side wall portion 22 is connected to the supporting plate portion 21.

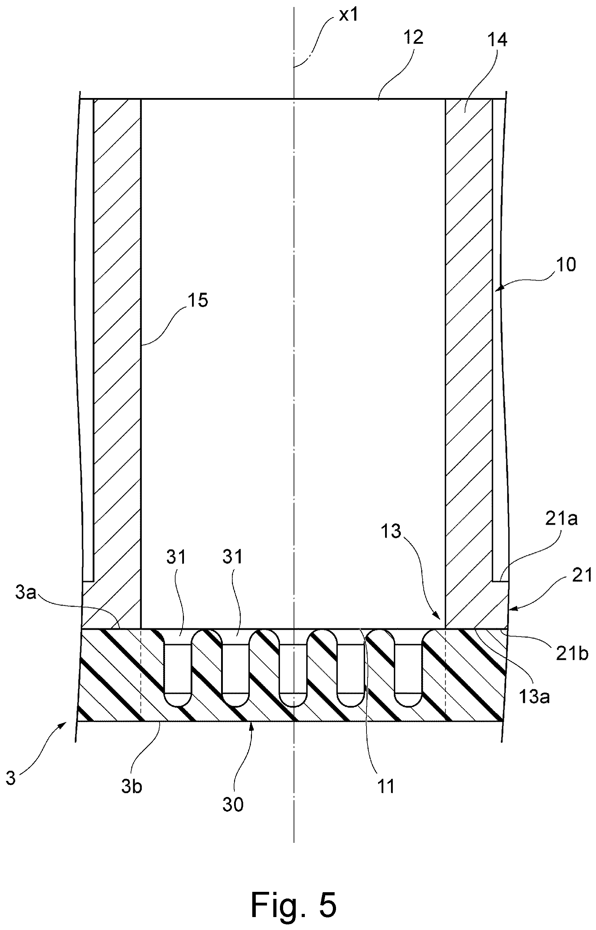

Figure 5 is a sectional view illustrating, enlarged, one tubular portion 10 and a vicinity thereof illustrated in Figure 4. As illustrated in Figure 5, the tubular portion 10 is a portion that forms a tubular surface 15 extending along an axial line x1 and forms a pair of openings 11 and 12. The tubular surface 11 defines a space that extends along an axial line x. The openings 11 and 12 are formed at a pair of end portions 13 and 14 in a direction of the axial line x1 of the tubular portion 10, respectively. For example, the tubular portion 10 is a cylinder or an approximate cylinder with the axial line x1 as a central axis or an approximately central axis. In addition, for example, the tubular surface 15 is a cylindrical surface or an approximately cylindrical surface with the axial line x1 as a central axis or an approximately central axis. The tubular portion 10 is connected to the supporting plate portion 21 on an outer circumferential side and, for example, as illustrated in Figure 5, the tubular portion 10 is connected to the supporting plate portion 21 at the end portion 13. An end surface 13a of the end portion 13 is flush with, for example, the lower surface 21b of the supporting plate portion 21. As illustrated in Figures 4 and 5, each of the plurality of tubular portions 10 extends in a same or approximately same direction, and the plurality of tubular portions 10 are supported by the supporting plate portion 21 in an aligned state. Note that the tubular portions 10 may be connected to the supporting plate portion 21 in a portion other than the end portion 13. For example, the tubular portions 10 may be connected to the supporting plate portion 21 in the end portion 14 or the tubular portions 10 may be connected to the supporting plate portion 21 in a portion between the end portion 13 and the end portion 14. Alternatively, for example, the tubular portions 10 may be connected to the supporting plate portion 21 in a portion ranging from a position between the end portion 13 and the end portion 14 to the end portion 13 or a portion ranging from a position between the end portion 13 and the end portion 14 to the end portion 14. Alternatively, the tubular portions 10 may be connected to the supporting plate portion 21 over an entire length between the end portion 13 and the end portion 14. In this case, the tubular portions 10 are to be included in the supporting plate portion 21.

The rigid member 2 is integrally formed from a same material, the tubular portions 10, the supporting plate portion 21, and the side wall portion 22 are each of portions of the integrally-formed rigid member 2, and the tubular portions 10, the supporting plate portion 21, and the side wall portion 22 are connected to each other. The material of the rigid member 2 is a material that prevents the rigid member 2 from deforming when the culture vessel 1 is grasped for handling. For example, a flexural modulus of the material of the rigid member 2 is 500 MPa or more. Alternatively, the flexural modulus of the material of the rigid member 2 is 1000 MPa or more. Alternatively, the flexural modulus of the material of the rigid member 2 is 1500 MPa or more. In addition, for example, the material of the rigid member 2 is a polystyrene resin. Note that the material of the rigid member 2 may be another resin material or a material that is not a resin material. As the resin material for the rigid member 2, in addition to polystyrene (PS), acrylic resin (PMMA), polypropylene (PP), cyclic olefin copolymer (COC), cyclic olefin polymer (COP), polycarbonate (PC), polymethylpentene (PMP), polyethylene (PE), and the like may also be used.

As described above, the bottom member 3 is a member having oxygen supply performance for culturing the object to be cultured. In other words, the bottom member 3 is a member capable of supplying oxygen required for culturing the object to be cultured. In the present example, the object to be cultured is spheroids and the bottom member 3 requires high oxygen supply performance. Therefore, for example, the bottom member 3 is formed from a silicone resin and the bottom member 3 has high oxygen permeability. For example, the silicone resin of the bottom member 3 is polydimethylsiloxane (poly(dimethylsiloxane) (PDMS)), a silicone hydrogel, or the like. When the culture vessel 1 is used for culturing cells that do not require large amounts of oxygen, in addition to using resin materials other than PDMS, such as polymethylpentene (PMP), polystyrene (PS), acrylic resin (PMMA), polypropylene (PP), cyclic olefin copolymer (COC), cyclic olefin polymer (COP), and polycarbonate (PC), glass and the like can also be used. In this manner, as long the material of the bottom member 3 has oxygen supply performance for culturing the object to be cultured, the material is not limited to silicone resin and may be other resins or non-resin materials. In addition, an oxygen permeation coefficient of the material of the bottom member 3 is, for example, 20 × 10⁻¹³ cm³ (STP) × cm/(cm² × s × Pa) or more. Alternatively, the oxygen permeation coefficient of the material of the bottom member 3 is, for example, 100 × 10⁻¹³ cm³ (STP) × cm/(cm² × s × Pa) or more. When the oxygen permeation coefficient of the material of the bottom member 3 is 20 × 10⁻¹³ cm³ (STP) × cm/(cm² × s × Pa) or more, oxygen for culturing cells can conceivably be supplied. When the oxygen permeation coefficient of the material of the bottom member 3 is 100 × 10⁻¹³ cm³ (STP) × cm/(cm² × s × Pa) or more, culturing of liver cells with high oxygen consumption is enabled.

As illustrated in Figures 2, 4, and 5, the bottom member 3 is a member that spreads in a plate shape and has a pair of surfaces 3a and 3b. In addition, as illustrated in Figures 2 and 5, the bottom member 3 is a member that spreads so as to cover an entirety of the plurality of tubular portions 10 from a side of the opening 11. The surface 3a and the surface 3b of the bottom member 3 are, for example, flat surfaces or approximately flat surfaces and are parallel or approximately parallel to each other. Furthermore, as illustrated in Figure 2, for example, the bottom member 3 contacts the side wall portion 22 of the support portion 20 on an outer circumferential side. The bottom member 3 is attached to the rigid member 2 and fixed to the rigid member 2.

As illustrated in Figure 5, the bottom member 3 closes one opening 11 of each of the plurality of tubular portions 10. Specifically, the surface 3a of the bottom member 3 contacts the end surface 13a of the end portion 13 of each tubular portion 10 over an entire periphery of the end surface 13a. In addition, as illustrated in Figure 5, a plurality of recessed portions 31 are formed on a bottom surface portion 30 being a portion of the bottom member 3 that closes the opening 11 of each tubular portion 10. The recessed portions 31 are recessed inward from a well bottom surface 32 that is a portion of the bottom surface portion 30 of the surface 3a of the bottom member 3. The well bottom surface 32 is a surface that closes the opening 11 of each tubular portion 10.

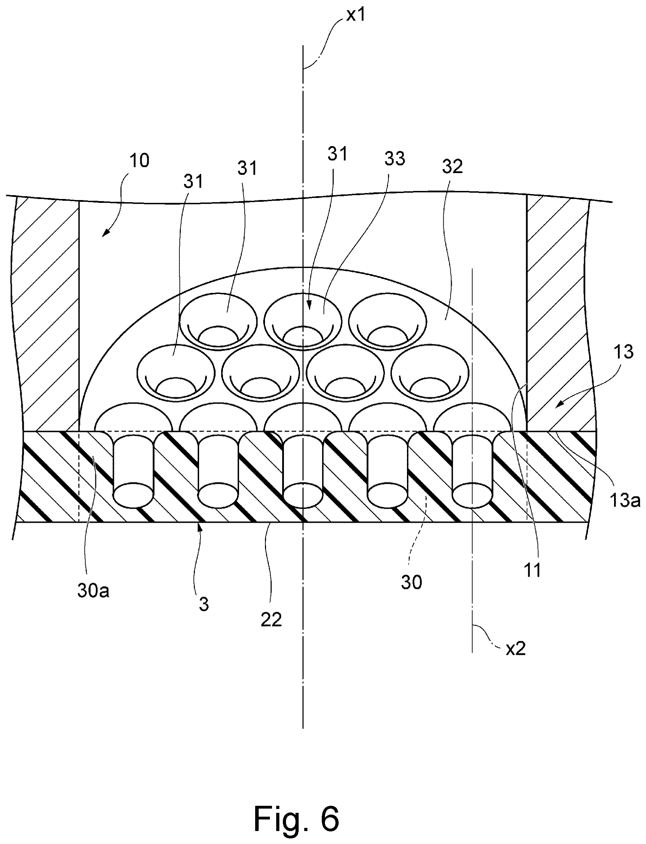

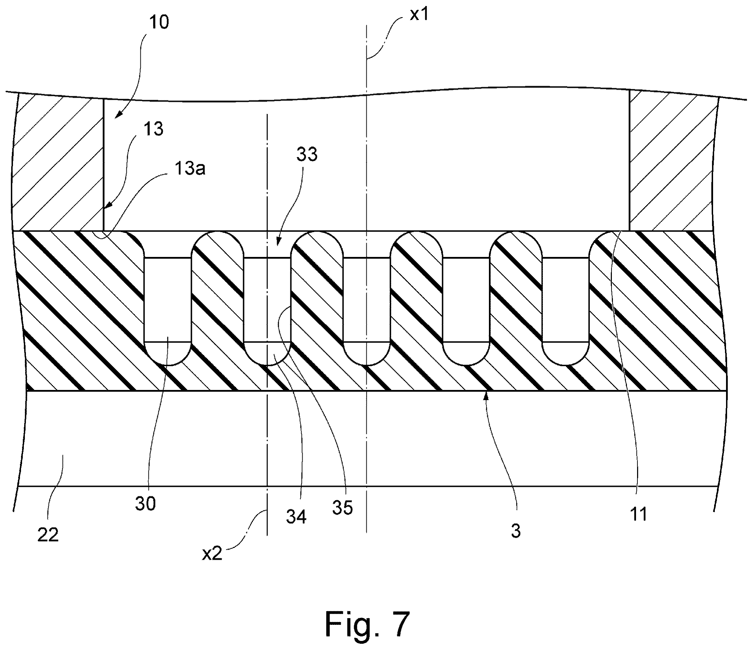

Figure 6 is a sectional perspective view illustrating, enlarged, a vicinity of the bottom surface portion 30 of the bottom member 3, and Figure 7 is a sectional view illustrating a cross section of the bottom surface portion 30 taken along a plane including the axial line x1. As illustrated in Figure 6, on the bottom surface portion 30, for example, the plurality of recessed portions 31 are formed inside an annular portion (peripheral edge portion 30a) adjacent to the end portion 13 of the tubular portion 10. The plurality of recessed portions 31 are formed in a region inside the peripheral edge portion 30a of the bottom surface portion 30. For example, the plurality of recessed portions 31 are formed in the region inside the peripheral edge portion 30a of the bottom surface portion 30 so that the plurality of recessed portions 31 are closest-packed. The recessed portions 31 extend along an axial line x2 and, for example, extend in a tubular surface shape with the axial line x2 as a central axis or an approximately central axis. For example, the axial line x2 is orthogonal or approximately orthogonal to the well bottom surface 32.

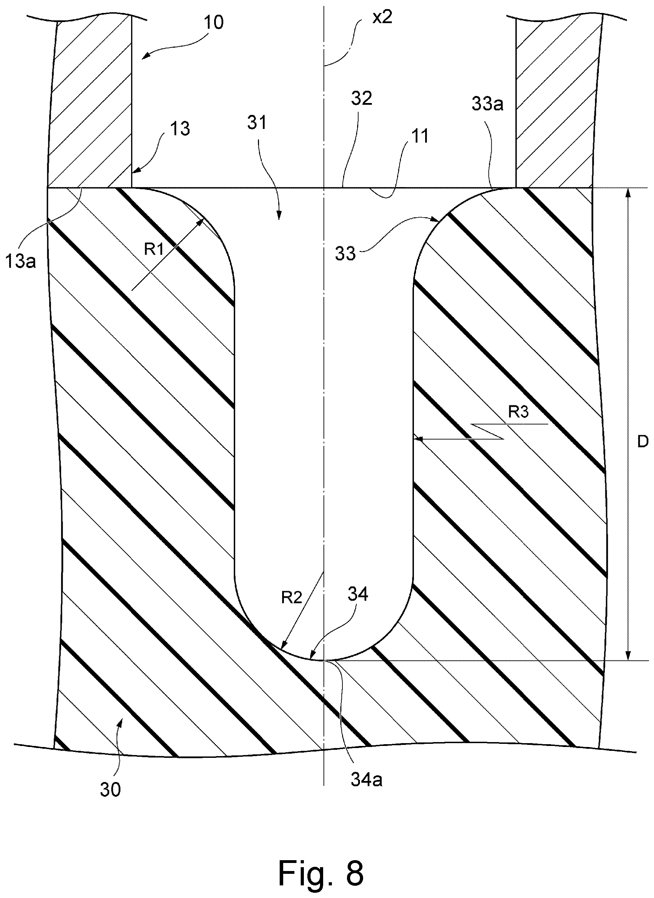

Figure 8 is a sectional view illustrating, enlarged, the recessed portion 31 illustrated in Figure 7 and is a sectional view illustrating a cross section of the bottom surface portion 30 taken along a plane including the axial line x2. As illustrated in Figures 6 to 8, for example, an opening 33 of the recessed portion 31 reduces in diameter toward a bottom 34 of the recessed portion 31. The opening 33 is an end portion on the side of the well bottom surface 32 of the recessed portion 31. The recessed portion 31 communicates with a space defined by the tubular portion 10 at the opening 33. A cross section of the opening 33 taken along a plane along a recess direction of the recessed portion 31 draws an arc as illustrated in, for example, Figure 8. Note that the recess direction of the recessed portion 31 is an extending direction of the recessed portion 31 and is a direction along the axial line x2. The opening 33 of the recessed portion 31 is, for example, an R curved surface. Specifically, for example, a contour on the cross section of the opening 33 draws a curved line with a constant or approximately constant radius of curvature R1 as illustrated in Figure 8.

As illustrated in Figures 6 to 8, the bottom 34 of the recessed portion 31 forms a surface along a spherical surface. Note that the bottom 34 is an end portion on an opposite side to the well bottom surface 32 of the recessed portion 31. The recessed portion 31 is closed at the bottom 34. For example, as illustrated in Figures 6 to 8, the bottom 34 forms a semispherical surface or an approximately semispherical surface. Specifically, for example, a contour in the cross section of the bottom 34 draws a curved line with a constant or approximately constant radius of curvature R2 as illustrated in Figure 8. The bottom 34 of the recessed portion 31 is not limited to forming a semispherical surface and may form surfaces of other shapes that form a space inside such as a conical surface or a pyramidal surface. Alternatively, the bottom 34 of the recessed portion 31 may be a flat surface or an approximately flat surface.

As illustrated in Figures 6 to 8, a middle 35 that is a portion between the opening 33 and the bottom 34 of the recessed portion 31 is a tubular surface. For example, as illustrated in Figure 8, in a cross section taken along a plane including the axial line x2, the middle 35 has a shape that draws a curve that is convex toward inside the recessed portion 31. For example, a diameter of the middle 35 decreases and then increases from a certain position when moving toward a side of the bottom 34 from a side of the opening 33. Specifically, for example, as illustrated in Figure 8, in a cross section taken along a plane including the axial line x2, the middle 35 has a shape that draws a curve with a constant or an approximately constant radius of curvature R3. The diameter of the middle 35 may decrease when moving toward the side of the bottom 34 from the side of the opening 33 or may increase when moving toward the side of the bottom 34 from the side of the opening 33.

Alternatively, in a cross section taken along a plane including the axial line x2, the middle 35 may have a shape that draws a curve that is convex toward outside the recessed portion 31. For example, the diameter of the middle 35 increases and then decreases from a certain position when moving toward the side of the bottom 34 from the side of the opening 33. Specifically, for example, in a cross section taken along a plane including the axial line x2, the middle 35 has a shape that draws a curve with a constant or an approximately constant radius of curvature R3. The diameter of the middle 35 may decrease when moving toward the side of the bottom 34 from the side of the opening 33 or may increase when moving toward the side of the bottom 34 from the side of the opening 33.

In addition, the middle 35 may form a cylindrical surface or an approximately cylindrical surface with the axial line x2 as a central axis or an approximately central axis. Alternatively, on a cross section taken along a plane including the axial line x2, the middle 35 may draw a curve that differs from the curve described above, a straight line, or a line that combines a curve and a straight line.

A depth D of the recessed portion 31 is set to a value which, for example, prevents cells or spheroids that are objects to be cultured from popping out of the recessed portion 31 when replacing a culture fluid and which makes it easy to design the culture vessel 1 so as to have a shape that conforms to the specifications of a well plate. As illustrated in Figure 8, the depth D of the recessed portion 31 is a distance of the recessed portion 31 in a direction of the axial line x2 and is a distance between an end 33a on a side of the well surface 32 of the opening 33 and an end 34a on an opposite side to the well surface 32 of the bottom 34. Specifically, for example, the depth D of the recessed portion 31 is 0.5 mm or more and 2.0 mm or less. The depth D of the recessed portion 31 may be another value.

The well bottom surface 32 is, for example, a flat surface or an approximately flat surface. Note that the well bottom surface 32 need not be a flat surface. For example, the axial line x2 of the recessed portion 31 is orthogonal or approximately orthogonal to the well bottom surface 32. The axial line x2 of the recessed portion 31 need not be orthogonal to the well bottom surface 32.

As illustrated in Figure 6, for example, the opening 33 of one recessed portion 31 contacts the opening 33 of another recessed portion 31 that is in proximity. Specifically, the end 33a of the opening 33 of the one recessed portion 31 contacts the end 33a of the opening 33 of another recessed portion 31 that is adjacent to the one recessed portion 31 via a thin-walled portion of the bottom surface portion 31. Specifically, for example, the end portion 33a of the opening 33 of one recessed portion 31 contacts the end portion 33a of the opening 33 of all recessed portions 31 in proximity to the opening 33. Accordingly, the plurality of recessed portions 31 are closest-packed in the region inside the peripheral edge portion 30a of the well bottom surface 32.

The culture vessel 1 is configured as described above and is used to, for example, culture spheroids. Specifically, when cells of the object to be cultured are seeded into each tubular portion 10 of the culture vessel 1, the seeded cells are accommodated in the plurality of recessed portions 31 formed in the bottom surface portion 30 of the bottom member 3 made of PDMS. The cells accommodated in the recessed portions 31 aggregate and form spheroids.

As described above, the bottom member 3 on which spheroids are to be cultured is created from PDMS with superior oxygen permeability. Therefore, oxygen supply performance to inside the recessed portions 31 where cells that are objects to be cultured are accommodated is increased. Accordingly, when culturing spheroids, sufficient oxygen for culturing the spheroids can be supplied to inside the recessed portions 31 where cells are accommodated. In this manner, the bottom member 3 of the culture vessel 1 has high oxygen supply performance to the recessed portions 31.

A member made of PDMS is flexible and readily deformable. Therefore, when an object made of PDMS is grasped for handling, the object made of PDMS deforms or may deform. Using PDMS as a material for an object that may cause inconvenience due to deformation when grasped may make handling of the object difficult. When the culture vessel deforms when handled, cells may scatter, and it may become difficult to fix the culture vessel for microscopic observation. Therefore, a culture vessel that deforms when grasped may cause inconvenience and a culture vessel made of PDMS may turn out to be an object that is difficult to handle. On the other hand, in the culture vessel 1, while the bottom member 3 is made of PDMS and the bottom member 3 is readily deformable, the tubular portions 10 and the support portion 20 constitute the rigid member 2 and the bottom member 3 is attached to the rigid member 2. Therefore, the culture vessel 1 does not deform when the culture vessel 1 is grasped in order to handle the culture vessel 1. Since the tubular portions 10 and the support portion 20 occupy a major part of the portion that maintains the outer shape of the culture vessel 1, the culture vessel 1 is less likely to deform when grasped despite having the bottom member 3 made from PDMS. In this manner, even if the culture vessel 1 has the flexible bottom member 3 made from PDMS that is capable of supplying sufficient oxygen for culturing spheroids, the culture vessel 1 is less likely to deform and the culture vessel 1 is easy to handle.

When the surface 3a of the bottom member 3 is a flat surface or an approximately flat surface, a region of the bottom member 3 which the end surface 13a of the end portion 13 of the tubular portions 10 contacts can be increased, and positioning of the bottom member 3 with respect to the tubular portions 10 can be made easy. Therefore, when the surface 3a of the bottom member 3 is a flat surface or an approximately flat surface, attaching of the bottom member 3 to the tubular portions 10 can be made easy.

In addition, the plurality of recessed portions 31 are formed in the bottom surface portion 30 of the bottom member 30 that forms a bottom of each tubular portion 10 of the culture vessel 1 and spheroids can be cultured in each recessed portion 31. Therefore, according to the culture vessel 1, a plurality of spheroids can be cultured at the same time and culturing efficiency of spheroids can be improved. In addition, the plurality of recessed portions 31 are closest-packed in the bottom surface portion 30 of the bottom member 30 and, even in this respect, the culture vessel 1 can improve the culturing efficiency of spheroids.

Furthermore, the opening 33 of the recessed portion 31 of the bottom surface portion 30 reduces in diameter toward the bottom 34. Accordingly, an area of the well bottom surface 32 of the bottom surface portion 30 is reduced and a proportion of a flat surface of the surface of the bottom surface portion 30 contacting the space inside the tubular portions 10 is reduced. In addition, since the opening 33 increases in diameter toward the well bottom surface 32, an entrance to the recessed portions 31 is wide. The entrance to the recessed portions 31 is wide. Therefore, cells seeded in the tubular portions 10 can readily enter the recessed portions 31 and the number of cells remaining in the well bottom surface 32 can be reduced. Accordingly, variability in the number and the size of spheroids formed in each recessed portion 31 can be reduced. Furthermore, since the opening 33 of the recessed portion 31 reduces in diameter toward the bottom 34, a surface area of the recessed portion 31 can be increased and, even in this respect, the culture vessel 1 can improve the oxygen supply performance into the recessed portion 31.

When the middle 35 of the recessed portion 31 has a shape in which the contour of the middle 35 of the recessed portion 31 in a cross section taken along a plane including the axial line x2 draws a curve, a surface area of the middle 35 can be increased. Therefore, even in this respect, the culture vessel 1 can improve the oxygen supply performance into the recessed portion 31.

In addition, the bottom member 3 has a plate shape and enables the recessed portions 31 to be readily formed. Furthermore, since the recessed portions 31 with a depth that prevents cells and spheroids accommodated in the recessed portions 31 from popping out of the recessed portions 31 can be formed in the bottom member 3, it is possible to prevent cells and spheroids from popping out of the recessed portions 31 in operations of the culture vessel 1 during culturing such as medium replacement, thereby facilitating operation of the culture vessel 1. In addition, since the culture vessel 1 can be given a shape that conforms to specifications of a well plate, the culture vessel 1 can also be used in apparatuses and the like that use conventional well plates.

As described above, the culture vessel 1 according to the embodiment of the present invention enables oxygen supply performance to be increased and handling to be made easier.

While the present invention has been described through the embodiment described above, it should be noted that the technical scope of the present invention is not limited to the scope described in the embodiment. It will be apparent to those skilled in the art that various changes and modifications may be made to the above embodiment. It is clear from the description of the claims that such modifications or improvements are also included within the technical scope of the present invention.

It should be noted that the embodiment described above is for facilitating understanding of the present invention and is not intended to limit the interpretation of the present invention. In addition, the embodiment described above is not intended to limit use objects in which the present invention is to be used and the present invention can include everything as use objects. The respective constituent elements included in the embodiment described above and arrangements, materials, conditions, shapes, sizes and the like of such constituent elements are not limited to those exemplified and can be modified as appropriate. For example, the present invention includes differences that occur in implementation such as manufacturing tolerances. Furthermore, constituent elements shown in different embodiments can be partially replaced or combined within a technically consistent range. In addition, each component can be selectively combined as appropriate so as to achieve at least a part of the objects and advantageous effects described above.

For example, although only the bottom member 3 is formed by PDMS in the culture vessel 1, other portions of the culture vessel 1 may also be made of PDMS as long as inconvenience due to deformation does not occur when grasped for handling. For example, the tubular portions 10 may be made of PDMS. As described above, while members made from silicone resins such as PDMS are materials with high oxygen permeability, the materials are prone to drug adsorption and absorption, which may affect the concentration of drugs in the culture fluid. Therefore, portions to be made of PDMS may be selected by also taking an effect on the concentration of drugs in the culture fluid into consideration. This also applies to cases where the bottom member 3 is made of a material that is not PDMS.

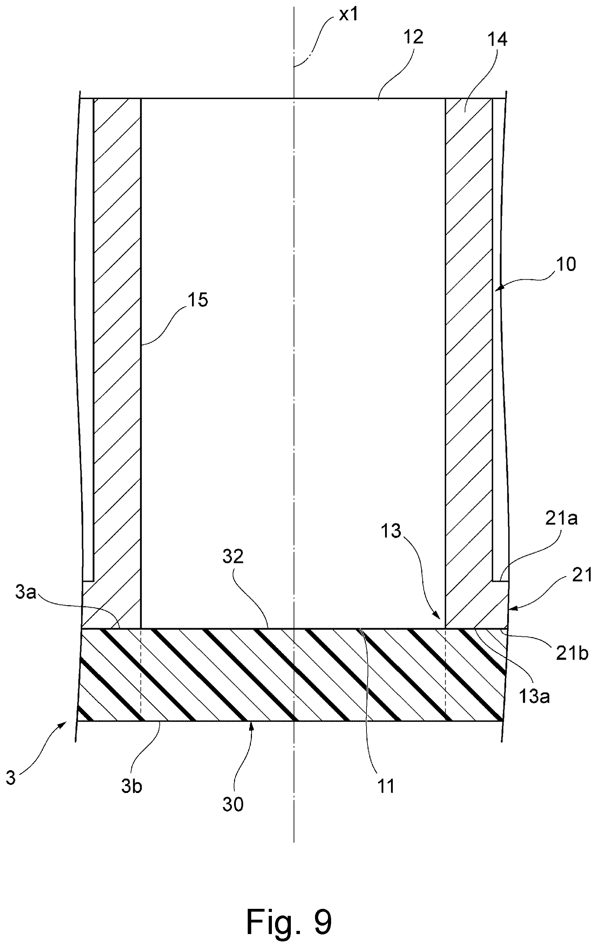

In addition, for example, as illustrated in Figure 9, the well bottom surface 32 of the bottom surface portion 30 of the bottom member 3 may be a smooth surface. In other words, the recessed portions 31 need not be formed in the bottom surface portion 30 and the well bottom surface 32 may be a smooth surface as illustrated in Figure 9. The well bottom surface 32 is, for example, a flat surface or an approximately flat surface that is parallel or approximately parallel to a plane that is orthogonal to the axial line x1. Alternatively, for example, the well bottom surface 32 may be a surface along a spherical surface and specifically, for example, a semispherical surface or an approximately semispherical surface. Alternatively, the well bottom surface 32 may be a surface such as a conical surface or a pyramidal surface or surfaces with other shapes.

### Reference Signs List

1 culture vessel, 2 rigid member, 3 bottom member, 10 tubular portion, 11, 12 opening, 13, 14 end portion, 13a end surface, 15 tubular surface, 20 support portion, 21 supporting plate portion, 21a upper surface, 21b lower surface, 22 side wall portion, 30 bottom surface portion, 30a peripheral edge portion, 31 recessed portion, 32 well bottom surface, 33 opening, 33a end, 34 bottom, 34a end, 35 middle, R1, R2, R3 radius of curvature, x1, x2 axial line

## Claims

1. A culture vessel, comprising:
a member that does not deform when grasped for handling; and
a bottom member that is a member having oxygen supply performance for culturing an object to be cultured.

2. The culture vessel according to claim 1, wherein
an oxygen permeation coefficient of the bottom member is 20 × 10⁻¹³ cm³ (STP) × cm/(cm² × s × Pa) or more.

3. The culture vessel according to claim 1, wherein
the bottom member is formed from a silicone resin.

4. The culture vessel according to any one of claims 1 to 3, wherein
a flexural modulus of the member that does not deform is 500 MPa or more.

5. The culture vessel according to claim 1, wherein
the member that does not deform is formed from a hard resin.

6. The culture vessel according to claim 1, wherein
the bottom member is attached to the member that does not deform.

7. The culture vessel according to claim 6, comprising:
a plurality of tubular portions being portions that have a tubular shape; and
a support portion being a portion that supports the plurality of tubular portions, wherein
the member that does not deform includes at least the support portion among the plurality of tubular portions and the support portion, and
the bottom member closes one opening of each of the plurality of tubular portions.

8. The culture vessel according to claim 7, wherein
a bottom surface portion being a portion of the bottom member that closes the one opening of each of the plurality of tubular portions has a smooth surface that closes the opening.

9. The culture vessel according to claim 7, wherein
a plurality of recessed portions are formed on the bottom surface portion being a portion of the bottom member that closes the one opening of each of the plurality of tubular portions.

10. The culture vessel according to claim 9, wherein
an opening of each recessed portion reduces in diameter toward a bottom of the recessed portion.

11. The culture vessel according to claim 10, wherein
a cross section of the opening taken along a surface along a recess direction of the recessed portion draws an arc.

12. The culture vessel according to claim 10, wherein
a bottom of the recessed portion forms a surface along a spherical surface.

13. The culture vessel according to claim 10, wherein
in the bottom surface portion, the opening of one of the recessed portions contacts the opening of another recessed portion in proximity.

14. The culture vessel according to claim 10, wherein
a space between the opening and the bottom of the recessed portion is a tubular surface.

15. The culture vessel according to claim 10, wherein
a depth of the recessed portion is 0.5 mm or more and 2.0 mm or less.

16. The culture vessel according to claim 7, wherein
the member that does not deform includes the plurality of tubular portions and the support portion.

17. The culture vessel according to claim 3, wherein
the silicone resin is polydimethylsiloxane.

18. The culture vessel according to claim 1, wherein
the culture vessel is to be used to culture spheroids, and
the object to be cultured is cells.
